# EUROPEAN PATENT SPECIFICATION

(11) **EP 1 191 908 B1**
(45) Date of publication and mention of the grant of the patent: **30.06.2004**
(21) Application number: 00946520.4
(22) Date of filing: 03.07.2000
(51) Int. Cl.: A61F 2/42

(54) **DISTAL RADIO-ULNAR JOINT PROSTHESIS**
DISTALE RADIO-ULNARE GELENKPROTHESE
PROTHESE DE L'ARTICULATION RADIO-CUBITALE DISTALE

(30) Priority: 02.07.1999 NL 1012491
(43) Date of publication of application: 03.04.2002
(73) Proprietor: van Straten Research & Development B.V., 3440 AK Nieuwegein (NL); Schuurman, Arnold Herman, 3645 JJ Vinkeveen (NL)
(72) Inventor: SCHUURMAN, Arnold, Herman, NL-3645 JJ Vinkeveen (NL)
(74) Representative: van Westenbrugge, Andries
(86) International application number: PCT/NL2000/000463
(87) International publication number: WO 2001/001892

(56) References cited:
- FR-A- 2 660 856
- GB-A- 1 412 376
- NL-C- 1 008 880
- US-A- 5 108 444
- US-A- 5 951 604

## Description

The invention relates to a distal radioulnar joint prosthesis (abbreviated as DRU joint prosthesis), comprising;
- a support;
- a ball joint; and
- an ulna shaft,
the one end of the ulna shaft and the support being joinable via the ball joint in a manner which allows them to hinge relative to one another. With this arrangement the other end of the ulna shaft will be intended to be attached to the distal end of the ulna and the support will be intended to be attached to the distal part of the radius.

A distal radioulnar joint prosthesis of this type is disclosed in the publication entitled "An Original Prosthesis of the Distal Radioulnar Joint" by Adalbert I. Kapandji (copy enclosed when filing this application). This publication shows a distal radioulnar joint prosthesis on page 330 in Figure 12. In order to fix this distal radioulnar joint prosthesis the distal ulna is removed and replaced by a prosthesis. The prosthesis comprises a support component that is screwed tightly to the side edge of the distal ulna. The ball of the ball joint is immovably integrated with this support. The prosthesis further consists of an ulna shaft, one end of which is inserted in the longitudinal direction into the ulna in order for the other end to bear on the latter. The ulna shaft inserted into the ulna is then fixed to the ulna by screwing. The socket of the ball joint is immovably integrated with the other end of the ulna shaft. In the implanted position, the socket and the ball engage in one another in order to permit rotation in three orthogonal directions. This DRU prosthesis from the article by Kapandji thus consists of two rigid components which, in turn, are rigidly fixed to, on the one hand, the distal radius and, on the other hand, the distal end of the ulna. In particular, shortening of the ulna is not possible. Lengthening of the ulna is at most possible if ball and socket are able to move apart, which, however, is at least associated with the risk of the ball permanently popping out of the socket.

In the natural position without a prosthesis the distal end of the ulna and the distal end of the radius are not rigidly coupled to one another. These ends are essentially located more or less separately alongside one another and the distal end of the smaller ulna is kept in place relative to the distal end of the radius by the triangular fibrocartilaginous complex. On moving the wrist and the hand the ulna and radius are thus able to execute fairly complex movements or. in other words, in the natural position ulna and radius have a high freedom of movement relative to one another. The ulna and radius can move relative to one another in the longitudinal direction. The ulna and radius can turn relative to one another, for example in the sense that the ulna and radius which in the initial position run parallel to one another come into a position in which they cross one another or in the sense that the ulna rotates about its longitudinal axis. The types of movement which the ulna and radius. in particular at the distal ends thereof, are able to undergo are known per se to those skilled in the art. In the case of the DRU joint prosthesis disclosed in the article by Kapandji the freedom of movement is appreciably restricted, in particular as a consequence of the mutually rigid fixing of the distal ends of ulna and radius.

The aim in the case of the so-called "Swanson ulnar head prosthesis" is to keep the freedom of movement of ulna and radius intact as far as possible by replacing the distal ulnar head by an ulnar head prosthesis and holding this, as it were, alongside the distal radius by means of an artificial fitted band. The relative movement of ulna and radius in the longitudinal direction can be maintained in this way. However, this Swanson ulnar head prosthesis again has the disadvantage that it is made of silicone and thus disintegrates in the course of time. Moreover, the DRU joint has no stability. FR-A-2 660 856 discloses the features of the preamble of claim 1.

The aim of the present invention is to provide a distal radioulnar joint prosthesis (DRU joint prosthesis) with which, on the one hand, the freedom of movement of the distal end of the ulna relative to the distal radius is kept intact as far as possible and with which, on the other hand, the distal end of the ulna and the distal end of the radius are mechanically fixed to one another.

This aim is achieved according to the invention with a distal radioulnar joint prosthesis of the type mentioned in the preamble, in that the ball of the ball joint is provided with a recess in which the one end of the ulna shaft can be accommodated such that it is slidable in the longitudinal direction thereof. The ball joint of the DRU joint prosthesis according to the invention thus has a translational freedom in the longitudinal direction of the ulna in addition to the rotational freedom in three orthogonal directions. In other respects the distal end of the radius and the ulna are mechanically fixed to one another, in particular in the lateral direction. In particular the translational freedom in the longitudinal direction of the ulna increases the freedom of movement of the wrist and the hand, so that the impedance on the freedom of movement as a consequence of the DRU joint prosthesis is appreciably, if not completely, eliminated.

In order to counteract lateral movement of the end of the ulna shaft accommodated in the recess, and thus wearing away of the recess and/or wear of the end of the ulna shaft accommodated in the recess, it is advantageous according to the invention if the one end of the ulna shaft can be accommodated transversely to the longitudinal direction thereof such that it fits in the recess. More or less uncontrolled lateral movement of the ulna shaft in the recess and wear caused as a result are then prevented. In order to make it possible for the ulna to make a translational movement relative to the radius without in so doing exerting a moment on the ball joint, it is advantageous according to the invention if the recess has a longitudinal axis which extends through the centre of the ball joint.

According to the invention it is particularly advantageous if the recess is a passage extending through the entire ball of the ball joint. This counteracts formation of a cavity in the ball joint, the volume of which cavity increases or decreases on translation of the ulna relative to the radius. Especially in the case of decrease in the volume, this would impede the translation of the ulna, since material present in this volume then has to be driven out.

Fitting, or to put it more accurately implantation, of the DRU joint prosthesis according to the invention can be appreciably facilitated if the recess is a passage extending through the entire ball of the ball joint and if the cross-section of the passage and of the ulna shaft are made such that the other end of the ulna shaft can be inserted in the passage and can then be inserted through the passage until the one end is in the passage. Thus, during implantation, after the support has been attached to the distal radius head the ulna shaft can first be inserted from the hand side of the support by its other end (which is intended to be accommodated in the ulna) into and through the passage until the one end (which is intended to be accommodated in the passage through the ball joint) is in the passage.

So that the ball of the ball joint can not pop out of the socket it is advantageous according to the invention if the ball is enclosed in the socket of the ball joint.

According to a further advantageous embodiment the other end of the ulna shaft will have been constructed such that it can be accommodated in the marrow cavity of the ulna and can be cemented therein, preferably using so-called bone cement, or can become fixed therein by in-growth by providing the other end of the ulna with a suitable coating, such as a hydroxyapatite coating.

According to a further advantageous embodiment of the invention the support will have been provided with a pin for cementing into the distal radius.

The present invention will now be explained in more detail below with reference to the drawings. In the drawings:
Figure 1 shows, diagrammatically, a plan view, partially in section. of a hand and wrist. showing the natural position of the distal radioulnar joint;
Figure 2 shows, diagrammatically, partially in plan view and partially in section, the fitting or implantation of the DRU joint according to the invention;
Figure 3 shows, diagrammatically, the DRU joint according to the invention in the implanted position, the freedoms of movement of the DRU joint prosthesis according to the invention also being indicated.

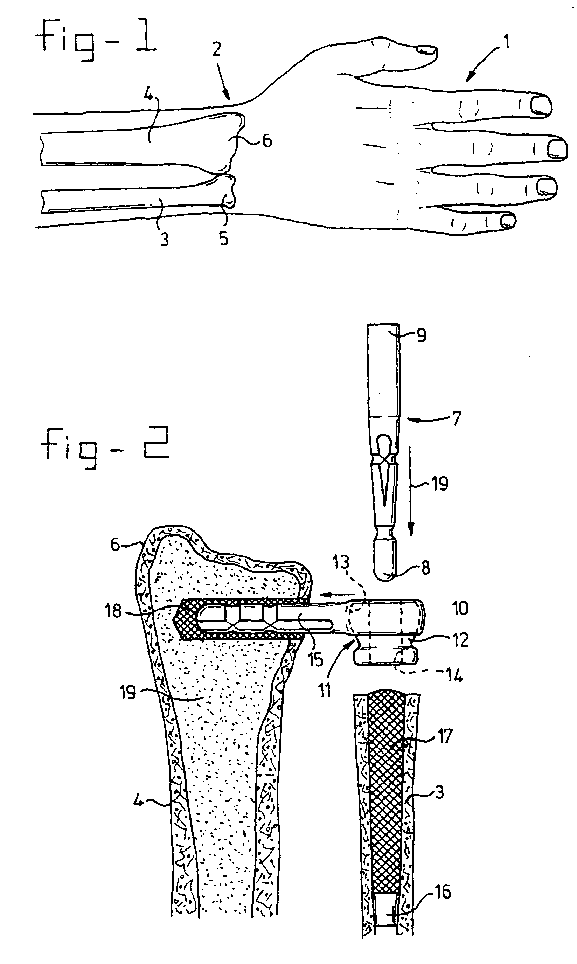

To define the concept, Figure 1 shows a hand 1, a wrist 2 and part of a lower arm, in which the distal end of the ulna 3 and the distal end of the radius 4 have been drawn. The distal ulnar head is indicated by 5 and the distal radius by 6. In the natural position the distal ulnar head 5 and distal radius 6 are located as it were alongside one another and are held in this position with respect to one another by the tissue, in particular relatively soft tissue. The distal ulnar head 5 and distal radius 6 are thus not mechanically fixed to one another.

There are a wide variety of reasons which can make it necessary to replace the ulnar head 5 by a so-called DRU prosthesis. The distal ulnar head 5 can have come into the incorrect position as a consequence of a fracture, can have been impaired as a consequence of disease, for example rheumatism, can display a congenital abnormality or can have been impaired or display an abnormality as a result of surgery or trauma. These, but also many other causes, such as, for example, irritation as a consequence of pressure exerted on the bones of the hand by the distal ulnar head, can give rise to implantation of a DRU prosthesis.

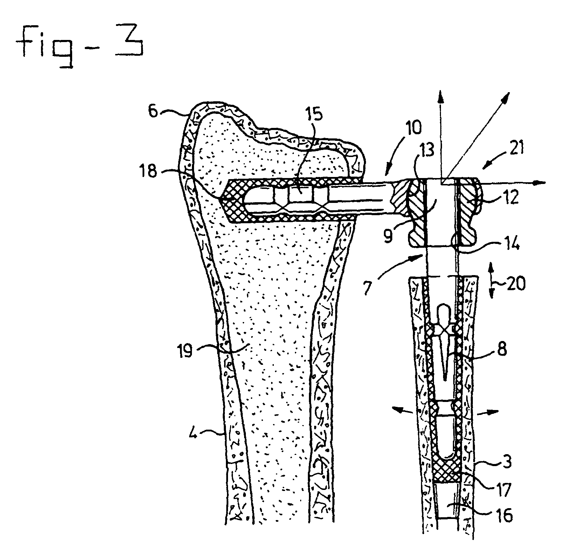

Figures 2 and 3 show, diagrammatically, a DRU joint prosthesis according to the invention, Figure 2 as it were illustrating the method of fitting and Figure 3 showing the fitted position.

The DRU joint prosthesis according to the invention consists of a support 10 which can be fixed laterally to the radius 6. This fixing could be effected by screwing the support 10 by means of a location onto the outside of the radius head (not shown). In the embodiment shown support 10 is, however. fixed to the radius 6 by making a hole in the latter, which hole is filled with bone cement 18 and into which hole a pin formed on the support 10 is inserted. Such a bone cement 18 sets relatively quickly, for example by means of polymerisation. For fixing the support 10 to the radius 6 it suffices merely to hold the support 10 still during polymerisation.

The support 10 is furthermore provided with a socket 13 in which a ball 12 is accommodated. As can be seen from Figure 3, this ball 12 is enclosed in the socket 13 so that the ball 12 is not able to detach from the socket 13 or to pop out of the latter. As is shown in Figure 3 by means of three orthogonal, double-headed arrows. the ball 12 of the ball joint 11 has three orthogonal freedoms of rotation.

As should be clear from Figures 2 and 3, the ball 12 is provided with a passage 14 extending from the one side to the other side, the middle of which passage 14 along the axis intersects the centre of the ball 12.

The DRU joint prosthesis according to the invention further comprises an ulna shaft 7, which has a so-called one end 9, hereinafter termed hinge end 9, and a so-called other end 8, hereinafter termed ulna end 8. The ulna end 8 is intended to be accommodated in the distal end of the ulna 3, as is shown in Figure 3. The hinge end 9 is intended to be accommodated in the passage 14 in the ball 12. The ulna end 8 is of reduced cross-section compared with the cross-section of the passage 14, so that the ulna shaft 7 can easily be inserted in accordance with arrow 18 (Figure 2) from the hand side through the passage 14 until the hinge end 9 is located in the passage 14 (Figure 3). As will be clear to a person skilled in the art, this appreciably facilitates fitting of the ulna shaft 7 in its correct position in the distal end 3 and the ball 12 of the ball joint 11, since the support 10 can first be fitted relatively easily and the ulna shaft 7 can then be inserted through the passage 14 into the distal end of the ulna 3 from the little finger side, which can be made adequately accessible during an operation. To anchor the ulna shaft 7 by the ulna end 8 in the ulna 3 the cavity delimited by the ulna 3 is filled from the distal end with a bone cement 17. To prevent the ulna end 8 dropping down too deeply into the ulna 3, both during implantation and later on, the bottom of the cavity in the ulna is closed off by means of a plug or bung 16. The material used for bone cement 17 can be the same as that used for bone cement 18. Such bone cement is again able to set relatively quickly, for example by means of polymerisation. It can suffice to hold still, on the one hand, the ulna 3 and, on the other hand, the ulna shaft 7 fixed to one another until adequate polymerisation has taken place.

Viewed in the transverse direction, the hinge end 9 is tightly accommodated in the passage 14. In this context tightly accommodated is understood to mean that movement of the hinge end 9 of the ulna pin 7 in the transverse direction of the ulna shaft 7 is prevented, whilst, on the other hand, movement of the ulna end 9 in the longitudinal direction of the ulna shaft 7, as is shown by double-headed arrow 20 in Figure 3, is not impeded, or at least is not prevented. The hinge end 9 of the ulna shaft will then be able to make a to and fro translational movement in the ball 12 in accordance with the double-headed arrow 20.

The DRU joint prosthesis according to the invention thus has four freedoms of movement, specifically three orthogonal freedoms of rotation. as shown by the set of double-headed arrows 21 in Figure 3, and a to and fro translational freedom. as shown by the double-headed arrow 20 in Figure 3. As a consequence of these four degrees of freedom, the freedom of movement of wrist and hand is barely impeded, compared with the natural position indicated in Figure 1, or at least is appreciably less impeded than in the case of comparable prostheses from the prior art, whilst the distal end of the ulna 3 and the distal end of the radius 6 are mechanically coupled. This mechanical coupling has the advantage that instabilities in the DRU joint are completely precluded.

Many variants of the DRU prosthesis according to the invention are conceivable within the scope of the claims. The other end 8 of the ulna shaft 7 can, for example, be fixed to the ulna by means of screws, for example if the marrow cavity is not accessible. Furthermore it is possible to fix the support 10 to the side of the distal radius. for example by screwing instead of cementing the pin 15 therein.

## Claims

1. Distal radioulnar joint prosthesis comprising:
- a support (10);
- a ball joint (11); and
- an ulna shaft (7),
the one end (9) of the ulna shaft (7) and the support (10) being joinable via the ball joint (11) in a manner which allows them to hinge relative to one another,
the other end (8) of the ulna shaft (7) being intended to be fixed to the distal end of the ulna (3); and
the support (10) being intended to be fixed to the distal part of the radius (6),
**characterised in that** the ball (12) of the ball joint (11) is provided with a recess (14) in which the one end (9) of the ulna shaft (7) can be accommodated such that it is slidable in the longitudinal direction (arrow 20) of the ulna shaft (7).

2. Joint prosthesis according to Claim 1, **characterised in that** the one end (9) of the ulna shaft (7) can be accommodated such that said one end of the ulna shaft, considered in a direction transversely to the longitudinal direction (arrow 20) of the shaft (7), fits tightly in the recess (14).

3. Joint prosthesis according to one of the preceding claims, **characterised in that** the recess (14) has a longitudinal axis which extends through the centre of the ball joint (11).

4. Joint prosthesis according to one of the preceding claims, **characterised in that** the recess (14) is a passage (14) extending through the entire ball (13) of the ball joint (11).

5. Joint prosthesis according to Claim 4, **characterised in that** the cross-section of the passage (14) and of the ulna shaft (7) are made such that the other end (8) of the ulna shaft (7) can be inserted in the passage (14) and can then be inserted through the passage (14) until the one end (9) is in the passage.

6. Joint prosthesis according to one of the preceding claims, **characterised in that** the ball (12) is enclosed in the socket (13) of the ball joint (11).

7. Joint prosthesis according to one of the preceding claims, **characterised in that** the other end (8) of the ulna shaft (7) is constructed such that it can be accommodated in the marrow cavity of the ulna (3) and preferably can be cemented therein or is able to grow together with the cavity by applying a suitable coating.

8. Joint prosthesis according to one of the preceding claims, **characterised in that** the support (10) is provided with a pin (15) for cementing into the distal (6) of the radius (4).

## Patentansprüche

1. Distale radioulnare Gelenkprothese mit:
einer Halterung (10);
einem Kugelgelenk (11); und
einem Ulnaschaft (7),
wobei das eine Ende (9) des Ulnaschafts (7) und die Halterung (10) über das Kugelgelenk (11) gelenkig miteinander verbunden werden können,
wobei das andere Ende (8) des Ulnaschafts (7) bestimmt ist, an dem distalen Ende der Ulna (3) befestigt zu sein; und
die Halterung (10) bestimmt ist, an dem distalen Teil des Radius (6) befestigt zu sein,
**dadurch gekennzeichnet, dass** die Kugel (12) des Kugelgelenks (11) mit einer Ausnehmung (14) versehen ist, in der das eine Ende (9) des Ulnaschafts (7) aufgenommen werden kann, so dass es in der Längsrichtung (Pfeil 20) des Ulnaschafts (7) verschiebbar ist.

2. Gelenkprothese gemäß Anspruch 1, **dadurch gekennzeichnet, dass** das eine Ende (9) des Ulnaschafts (7) derart aufgenommen werden kann, dass das eine Ende des Ulnaschafts, in einer Richtung quer zur Längsrichtung (Pfeil 20) der Welle (7) betrachtet, eng in die Ausnehmung (14) passt.

3. Gelenkprothese gemäß einem der vorhergehenden Ansprüche, **dadurch gekennzeichnet, dass** die Ausnehmung (14) eine Längsachse aufweist, die sich durch die Mitte des Kugelgelenks (11) erstreckt.

4. Gelenkprothese gemäß einem der vorhergehenden Ansprüche, **dadurch gekennzeichnet, dass** die Ausnehmung (14) ein Durchgang (14) ist, der sich durch die gesamte Kugel (13) des Kugelgelenks (11) erstreckt.

5. Gelenkprothese gemäß Anspruch 4, **dadurch gekennzeichnet, dass** der Querschnitt des Durchgangs (14) und des Ulnaschafts (7) so ausgeführt sind, dass das andere Ende (8) des Ulnaschafts (7) in den Durchgang (14) und dann durch den Durchgang (14) eingeführt werden kann, bis das eine Ende (9) in dem Durchgang ist.

6. Gelenkprothese gemäß einem der vorhergehenden Ansprüche, **dadurch gekennzeichnet, dass** die Kugel (12) in der Pfanne (13) des Kugelgelenks (11) eingeschlossen ist.

7. Gelenkprothese gemäß einem der vorhergehenden Ansprüche, **dadurch gekennzeichnet, dass** das andere Ende (8) des Ulnaschafts (7) so aufgebaut ist, dass es in der Knochenmarkhöhle der Ulna (3) untergebracht und vorzugsweise darin zementiert werden kann oder imstande ist mit dem Hohlraum durch Auftragen einer geeigneten Beschichtung zusammenzuwachsen.

8. Gelenkprothese gemäß einem der vorhergehenden Ansprüche, **dadurch gekennzeichnet, dass** die Halterung (10) mit einem Stift (15) zum Zementieren in den distalen Teil (6) des Radius (4) ausgestattet ist.

## Revendications

1. Prothèse d'articulation radio-cubitale distale comportant :
- un support (10) ;
- une rotule (11) ; et
- une tige cubitale (7),
la première extrémité (9) de la tige cubitale (7) et le support (10) pouvant être reliés par l'intermédiaire de la rotule (11) d'une manière qui leur permet d'être articulés l'un par rapport à l'autre,
l'autre extrémité (8) de la tige cubitale (7) étant prévue pour être fixée sur l'extrémité distale du cubitus (3) ; et
le support (10) étant prévu pour être fixé sur la partie distale du radius (6),
**caractérisée en ce que** la boule (12) de la rotule (11) est pourvue d'un renfoncement (14) dans lequel la première extrémité (9) de la tige cubitale (7) peut être reçue de telle sorte qu'elle peut coulisser dans la direction longitudinale (flèche 20) de la tige cubitale (7).

2. Prothèse d'articulation selon la revendication 1, **caractérisée en ce que** la première extrémité (9) de la tige cubitale (7) peut être reçue de telle sorte que ladite première extrémité de la tige cubitale, considérée dans une direction transversalement à la direction longitudinale (flèche 20) de la tige (7), s'ajuste de manière serrée dans le renfoncement (14).

3. Prothèse d'articulation selon l'une des revendications précédentes, **caractérisée en ce que** le renfoncement (14) a un axe longitudinal qui s'étend à travers le centre de la rotule (11).

4. Prothèse d'articulation selon l'une des revendications précédentes, **caractérisée en ce que** le renfoncement (14) est un passage (14) qui s'étend à travers toute la boule (13) de la rotule (11).

5. Prothèse d'articulation selon la revendication 4, **caractérisée en ce que** la section du renfoncement (14) et celle de la tige cubitale (7) sont rendues telles que l'autre extrémité (8) de la tige cubitale (7) peut être insérée dans le passage (14) et peut alors être insérée à travers le passage (14) jusqu'à ce que la première extrémité (9) soit dans le passage.

6. Prothèse d'articulation selon l'une des revendications précédentes, **caractérisée en ce que** la boule (12) est enfermée dans l'embase (13) de la rotule (11).

7. Prothèse d'articulation selon l'une des revendications précédentes, **caractérisée en ce que** l'autre extrémité (8) de la tige cubitale (7) est construite de telle sorte qu'elle peut être reçue dans la cavité médullaire du cubitus (3) et peut de préférence être collée dedans ou peut croître avec la cavité en appliquant un revêtement approprié.

8. Prothèse d'articulation selon l'une des revendications précédentes, **caractérisée en ce que** le support (10) est pourvu d'une broche (15) pour collage dans la partie distale (6) du radius (4).
